# EUROPEAN PATENT APPLICATION

(11) **EP 4 692 131 A1**
(43) Date of publication of application: **11.02.2026**
(21) Application number: 24784864.1
(22) Date of filing: 01.04.2024
(51) Int. Cl.: C08F 2/46, C08F 20/56, C09J 4/02, C09J 11/06, C09K 3/10

(54) **NOVEL ACRYLAMIDE, COMPOSITION CONTAINING SAME, CURABLE COMPOSITION, ADHESIVE, SEALING MATERIAL, CURED PRODUCT, SEMICONDUCTOR DEVICE, AND ELECTRONIC COMPONENT**

(30) Priority: 03.04.2023 JP 2023060069
(71) Applicant: Namics Corporation, Niigata-shi, Niigata 950-3131 (JP)
(72) Inventor: SATO, Ayako, Niigata-shi, Niigata 950-3131 (JP); IWAYA, Kazuki, Niigata-shi, Niigata 950-3131 (JP)
(74) Representative: Hoffmann Eitle
(86) International application number: PCT/JP2024/013411
(87) International publication number: WO 2024/210079

(57) **Abstract**

The task is to provide a new substance, a curable composition, an adhesive or sealant containing the new substance with excellent light curability and adhesive strength, a cured product in which the materials have been cured, and a semiconductor device or electronic component containing the cured product, which can solve the problems of bismaleimides having the above dimer acid skeleton.

Provided are dimer acid-modified acrylamide, compositions mainly containing dimer acid-modified acrylamide, curable compositions containing dimer acid-modified acrylamide and radical polymerization initiators, adhesives or sealants, cured products in which the curable composition above or the adhesive or sealant above has been cured, and semiconductor devices or electronic components containing such cured products.

## Description

### Field of the Invention

The present invention relates to new acrylamide, a composition containing the same, a curable composition, an adhesive, a sealant, a cured product, a semiconductor device and an electronic component.

### Background Art

Currently, in assembling and mounting parts, such as a semiconductor chip, used in a semiconductor device or an electronic component, an adhesive, a sealant and the like containing a curable resin composition are often used for such purposes as maintaining reliability.

Resin compositions containing polymaleimide, for example, are known as resin compositions used for such adhesives and sealants for electronic components. Polymaleimides having two or more maleimide groups are known to be photo- and thermo-curable by single polymerization such as radical polymerization or ionic polymerization of the double bond in the maleimide group, addition reaction with hydrogen of aromatic amines, copolymerization with allyl phenol, etc., to give cured products with excellent heat resistance, and to have good adhesive strength due to their high polarity. On the other hand, polymaleimide has high crystallinity, a high melting point, and low solubility in organic solvents, making it difficult to melt or dissolve to ensure formability when used as a curable composition. There is also concern that cured products of many commercially available polymaleimide have high water absorption and poor moisture reliability.

Patent Document 1 discloses an adhesive containing a bismaleimide compound with a dimer acid skeleton or the like introduced. Substances containing mainly bismaleimides having such a dimer acid skeleton are commercially available, such as BMI-689, BMI-1500, BMI-1700, etc. available from Designer molecules.

### PRIOR ART REFERENCE

### Patent Document

Patent Document 1: US 2010/0063184 A

### Summary of the Invention

### Problems to be Solved by the Invention

Bismaleimides having a dimer acid skeleton have reactivity derived from the maleimide group and can be polymerized by photoirradiation in the presence or absence of a photo-radical generator or by heating in the presence of a thermal radical generator. Bismaleimide with a dimer acid skeleton also has the advantage of providing a flexible (low modulus) cured product with excellent moisture resistance due to the dimer acid skeleton. On the other hand, bismaleimide with a dimer acid skeleton was found to have still insufficient curing depth and adhesive strength in light curing.

It is an object of the present invention to provide a new substance, a curable composition, an adhesive or sealant containing the new substance with excellent light curability and adhesive strength, a cured product in which the materials have been cured, and a semiconductor device or electronic component containing the cured product, which can solve the problems of bismaleimides having the above dimer acid skeleton.

### Means to solve the problem

Specific measures to solve the aforementioned problems are as follows.

Aspects of the present invention encompass the following aspects of new acrylamide, compositions primarily containing the acrylamide, curable compositions containing the same, adhesives or sealants, cured products, and semiconductor devices or electronic components.
[1] Dimer acid-modified acrylamide.
[2] A composition comprising
   dimer acid-modified acrylamide, and
   a medium, a polymerization inhibitor, or a combination thereof,
   wherein a content of the dimer acid-modified acrylamide in the composition is 83% by weight or more.
[3] A curable composition comprising
   dimer acid-modified acrylamide, and
   a radical polymerization initiator.
[4] The curable composition as described in [3] above, wherein the radical polymerization initiator is a photo-radical polymerization initiator.
[5] The curable composition as described in [3] above, wherein the radical polymerization initiator is a thermal radical polymerization initiator.
[6] The curable composition as described in any one of [3] to [5] above, further comprising a reactive diluent, a solvent, or a combination thereof.
[7] The curable composition as described in any one of [3] to [6] above, further comprising a curable component other than the dimer acid-modified acrylamide.
[8] The curable composition as described in any one of [3] to [7] above, which is configured as the dimer acid-modified acrylamide and the radical polymerization initiator being contained in a single container.
[9] The curable composition as described in any one of [3] to [7] above, which is configured as the dimer acid-modified acrylamide and the radical polymerization initiator being separated in two or more containers.
[10] An adhesive or sealant comprising the curable composition as described in any one of [3] to [9] above.
[11] A cured product in which the curable composition as described in any one of [3] to [9] above or the adhesive or sealant as described in [10] above has been cured.
[12] A semiconductor device or an electronic component comprising the cured product as described in [11] above.

### Effects of the Invention

According to the aspects of the present invention, new substances, curable compositions, adhesives or sealants containing the new substances with excellent light curability and adhesive strength, cured products in which the materials have been cured, and semiconductor devices or electronic components containing the cured products, which can solve the problems of bismaleimides having the above dimer acid skeleton, can be provided.

### Brief Description of Drawings

[Fig. 1] Fig. 1 shows ¹H NMR chart of dimer acid-modified acrylamide composition (A-4) of Production Example 4.
[Fig. 2] Fig. 2 shows ¹H NMR chart of dimer acid-modified acrylamide composition (A-5) of Production Example 5.
[Fig. 3] Fig. 3 shows an example of the mass spectrum of dimer acid-modified acrylamide composition (A-4) of Production Example 4.

### Mode for carrying out the Invention

### [New acrylamide and composition containing the same]

One aspect of the present invention is dimer acid-modified acrylamide.

Another aspect of the present invention is a composition comprising
dimer acid-modified acrylamide, and
a medium, a polymerization inhibitor, or a combination thereof,
wherein a content of the dimer acid-modified acrylamide in the composition is 83% by weight or more.

These aspects provide dimer acid-modified acrylamide and a dimer acid-modified acrylamide composition in liquid form that is substantially free of by-products (e.g., hydrogen halides and salts thereof) during synthesis of the dimer acid-modified acrylamide. With this dimer acid-modified acrylamide or dimer acid-modified acrylamide composition, it is possible to prepare a curable composition with excellent light-curable and adhesive properties, containing dimer acid-modified acrylamide in appropriate concentrations. In this specification, "substantially free of by-products" means that the amount of such by-products in the composition is less than 1% by weight.

Dimer acid is a liquid fatty acid that contains as its main component C36 dicarboxylic acid (dibasic acid) produced by dimerization of C18 unsaturated fatty acids from vegetable oils and fats, and can contain any amount of C18 unsaturated fatty acids (sometimes also called C18 monomeric acids), C54 trimeric acids, other polymerized fatty acids, etc. C18 unsaturated fatty acids include, for example, oleic acid, linoleic acid, linolenic acid, and the like. C36 dicarboxylic acids are mainly acyclic, monocyclic, polycyclic, and aromatic ring-type compounds represented by the structural formulas shown below. The C36 dicarboxylic acid in dimer acid can be a mixture containing these multiple C36 dicarboxylic acid compounds in any proportion, depending on the plant material and production method.

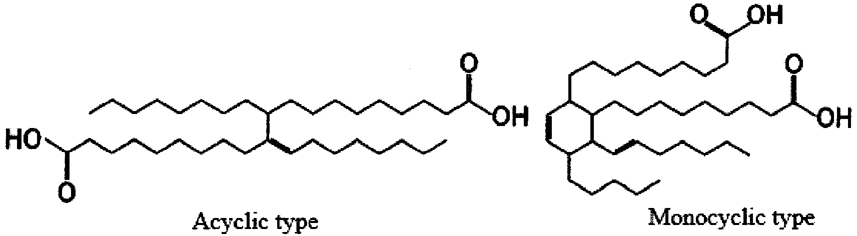

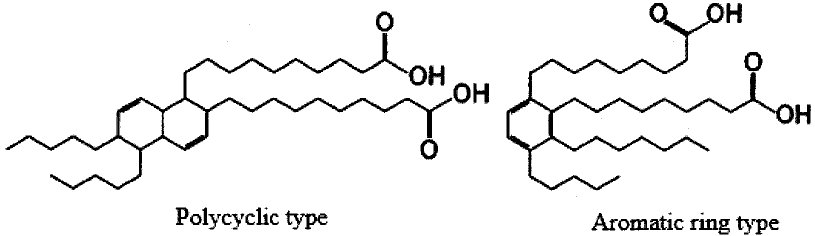

Although a double bond (C=C) remains in dimer acid after the polymerization reaction of unsaturated fatty acids, in this specification, hydrogenated products in which the degree of unsaturation is reduced by further hydrogenation reaction shall also be included in the definition of dimer acid. Dimer acid may also contain arbitrary amounts of C18 monomeric acid, C54 trimeric acid, and other polymerized fatty acids, depending on the production method, degree of purification, and other factors. As used herein, dimer acid may include those monomeric acids, trimeric acids, and other polymerized fatty acids.

As used herein, "dimer acid-modified acrylamide" can be defined as a substance in which the terminal carboxyl group (-COOH) of the above dimer acid has been replaced by a methylene acrylamide group (-CH₂-NH-CO-CH=CH₂), or can also be defined as a substance in which an acrylamide group (-NH-CO-CH=CH₂) is bonded to a hydrocarbon group derived from dimer acid. In other words, "dimer acid-modified acrylamide" herein refers to a substance that necessarily contains diacrylamide derived from C36 dicarboxylic acid as a major component and can contain (i.e., may or may not contain) monoacrylamide derived from C18 monomeric acid, triacrylamide derived from C54 trimeric acid, and any other polymerizable fatty acid-derived acrylamide in any amount.

As used herein, "hydrocarbon group derived from dimer acid" refers to a hydrocarbon group in which the terminal carboxyl group (-COOH) of dimer acid has been replaced by a methylene group (-CH₂-), also called "dimer acid skeleton".

Examples of diacrylamide in dimer acid-modified acrylamide include, but are not limited to, the followings:
Acyclic type represented by the following formulas: 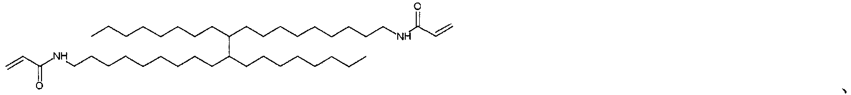 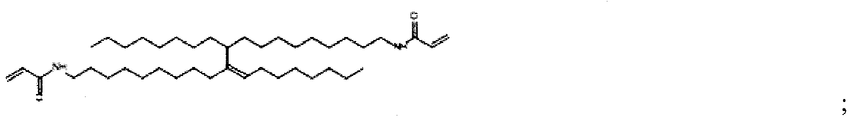
Monocyclic type represented by the following formulas: 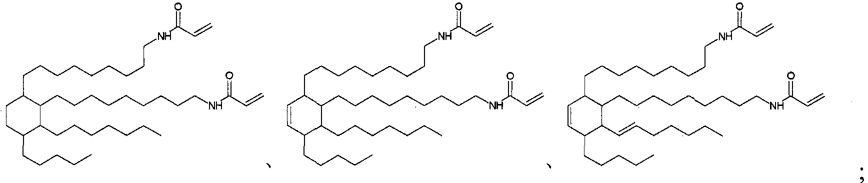
Polycyclic type represented by the following formulas: 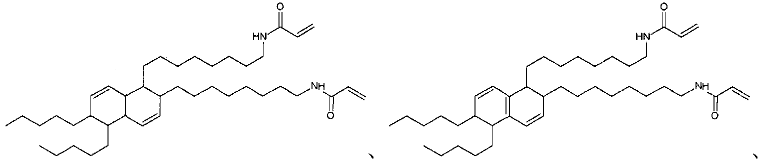 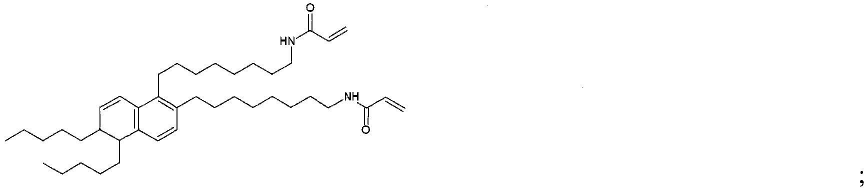 and
Aromatic ring type represented by the following formulas:

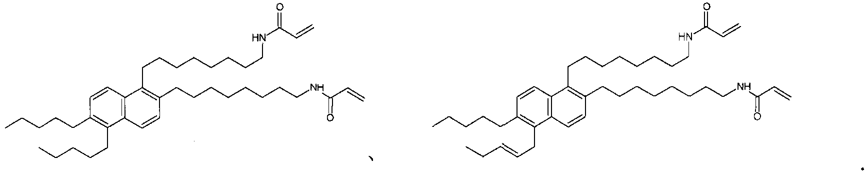

The content of diacrylamide, monoacrylamide, and triacrylamide in dimer acid-modified acrylamide depends on the raw materials and production/purification methods. In one embodiment, the contents of diacrylamide, monoacrylamide, and triacrylamide in dimer acid-modified acrylamide can be 75 to 100% by weight, 0 to 5% by weight, and 0 to 25% by weight, respectively. In another embodiment, the contents of diacrylamide, monoacrylamide, and triacrylamide in dimer acid-modified acrylamide can be 90 to 100% by weight, 0 to 5% by weight, and 0 to 10% by weight, respectively. In another embodiment, the contents of diacrylamide, monoacrylamide, and triacrylamide in dimer acid-modified acrylamide can be 95 to 100% by weight, 0 to 5% by weight, and 0 to 5% by weight, respectively. In another embodiment, the contents of diacrylamide, monoacrylamide, and triacrylamide in dimer acid-modified acrylamide can be 99 to 100% by weight, 0 to 1% by weight, and 0 to 1% by weight, respectively.

Methods for producing dimer acid-modified acrylamide are not particularly limited, and examples thereof include a method comprising reacting dimer diamine with an acrylic acid halide (acryloyl halide) in a solvent, in the presence or absence of a base.

Dimer diamine is a substance in which the terminal carboxyl group (-COOH) of dimer acid has been replaced with an aminomethyl group (-CH₂-NH₂), and can contain any amount of monoamine, triamine, etc. in addition to the main component diamine. The contents of monoamines and triamines in dimer diamine depend on the raw materials and the production/purification methods. Examples of commercial products of dimer diamine include, but are not limited to, the trade names "PRIAMINE 1071", "PRIAMINE 1073", "PRIAMINE 1074", and "PRIAMINE 1075" (all available from Croda Japan KK). For example, the above trade name "PRIAMINE 1071" contains about 75% by weight of diamine and about 25% by weight of triamine. For example, the above trade name "PRIAMINE 1075" contains more than 99% by weight of diamine and less than 1% by weight of triamine. See Polymer, Vol. 205 (2020), 122768.

Examples of acrylic acid halides (acryloyl halides) include acrylic acid chloride (acryloyl chloride) and acrylic acid bromide (acryloyl bromide).

The solvent can be any solvent that does not affect the reaction. Examples of the solvent include hydrocarbons (benzene, toluene, xylene, cyclohexane, etc.), non-protic polar solvents (N,N-dimethylformamide, dimethyl sulfoxide, N-methyl-2-pyrrolidone, etc.), nitriles (acetonitrile, etc.), ketones (acetone, methyl ethyl ketone, methyl isobutyl ketone, cyclohexanone, etc.), esters (ethyl acetate, butyl acetate, etc.), ethers (cyclopentyl methyl ether, diethyl ether, tetrahydrofuran, dimethoxyethane, etc.), halogenated solvents (dichloromethane, chloroform, etc.), and the like. These can also be used in appropriate combinations.

Examples of bases include, but are not limited to, triethylamine, pyridine, diisopropylethylamine, 2,6-lutidine, and 4-dimethylaminopyridine.

The reaction can be carried out at 0°C to 50°C, preferably 10°C to 30°C.

After the above reaction between dimer diamine and acrylic acid halide (acryloyl halide) is completed, the reaction is quenched. In this regard, it was found that the dimer acid-modified acrylamide produced is difficult to be separated and purified from the reaction mixture by usual liquid-liquid extraction methods because of its amphiphilic nature. In addition, heating under high vacuum for the purpose of removing solvent, which tends to remain due to high viscosity, sometimes resulted in the formation of a gel insoluble in chloroform. This is thought to be due to the unintentional generation of radicals in a low oxygen state, resulting in partial polymerization of dimer acid-modified acrylamide.

As a result of their diligent study, the inventors succeeded in separating dimer acid-modified acrylamide from the reaction mixture at a high concentration, to obtain a composition containing dimer acid-modified acrylamide and a medium, wherein the content of dimer acid-modified acrylamide in the composition is 83% by weight or more, which is one aspect of the present invention.

The inventors have also succeeded in preparing a dimer acid-modified acrylamide composition with further reduced amounts of medium or substantially free of medium by adding a polymerization inhibitor as appropriate during concentration. That is, another aspect of the present invention is a composition containing dimer acid-modified acrylamide and a polymerization inhibitor, wherein the content of dimer acid-modified acrylamide in the composition is 83% by weight or more. In one embodiment, the above dimer acid-modified acrylamide composition contains a medium and a polymerization inhibitor. In one embodiment, the above dimer acid-modified acrylamide composition contains a polymerization inhibitor and is substantially free of a medium. As used herein, "substantially free of medium" means that the amount of such medium in the composition is less than 0.5% by weight.

In the composition, the content of dimer acid-modified acrylamide is, for example, 83% by weight or more and less than 100% by weight, for example, 85% by weight or more and less than 100% by weight, for example, 90 to 99% by weight, for example, 93 to 98% by weight.

Methods for separating dimeric acid-modified acrylamide from the reaction mixture include, but are not limited to, the following methods.
(1) An acid such as hydrochloric acid and water are added to the reaction mixture, and the mixture is washed in separate batches to remove inorganic substances and amines that are byproducts. In this process, it is difficult to separate the layers because emulsions tend to form in the usual way. However, by adding inorganic salts such as sodium chloride, or by adding an appropriate organic solvent such as ethyl acetate, it is possible to separate the layers. The organic layer is separated and washed with an alkaline solution such as sodium hydroxide, followed by brine. The organic layer is dried with a drying agent such as anhydrous sodium sulfate, filtered, and concentrated in the presence or absence of a polymerization inhibitor to obtain a composition containing 83% by weight or more of the target product, dimer acid-modified acrylamide, and a medium. The medium can be the solvent used for the reaction, an organic solvent used for aliquoting after the reaction, water, a reactive diluent (see below for details), or a mixture thereof. The preferred viscosity of the medium is less than 1 Pa-s at 25°C.
(2) If necessary, the composition obtained in (1) above can be further concentrated to obtain a composition containing dimer acid-modified acrylamide in which the amount of medium in the composition is further reduced, or which is substantially free of medium. To reduce the risk of unintended polymerization reactions during the concentration operation, a polymerization inhibitor can be used as appropriate. For example, using only quinone-based or phenol-based polymerization inhibitors during vacuum concentration carries the risk of unexpected polymerization. Therefore, it is preferable to use an amine-based polymerization inhibitor such as N-nitroso-N-phenylhydroxylamine aluminum (NNAS) and phenothiazine, for example.

From the viewpoint of ease of handling the composition and the degree of freedom in designing curable compositions using the composition, the content of the medium in the composition is preferably from 1 to 17% by weight, more preferably from 1 to 15% by weight, and even more preferably from 2 to 8% by weight.

### [Curable composition]

One aspect of the present invention, a curable composition, contains
dimer acid modified acrylamide, and
a radical polymerization initiator.
According to this aspect, a curable composition with excellent light-curing properties and adhesive strength can be provided.

The dimer acid-modified acrylamide contained in the curable composition is the same as the dimer acid-modified acrylamide of the above aspect, so the description of dimer acid-modified acrylamide in the above aspect is also applicable to this aspect. The content of dimer acid-modified acrylamide in the curable composition is, for example, 1% by weight or more and less than 100% by weight, and for example, from 5 to 98% by weight.

Radical polymerization initiators include photo-radical and thermal radical polymerization initiators. In one embodiment, the radical polymerization initiator is a photo-radical polymerization initiator. In one embodiment, the radical polymerization initiator is a thermal radical polymerization initiator. In one embodiment, the radical polymerization initiator includes a photo-radical polymerization initiator and a thermal radical polymerization initiator.

The inclusion of a photo-radical polymerization initiator promotes UV curing of the curable composition. The type of photo-radical polymerization initiator is not limited and known materials can be used. Examples of photo-radical polymerization initiators include, but are not limited to, alkylphenone compounds and acylphosphine oxide compounds.

Examples of alkylphenone compounds include, but are not limited to, benzyl dimethyl ketal such as 2,2-dimethoxy-1,2-diphenylethan-1-one (commercially available as Omnirad 651 from IGM Resins B.V.); α-aminoalkylphenones such as 2-methyl-2-morpholino(4-thiomethylphenyl)propan-1-one (commercially available as Omnirad 907 from IGM Resins B.V.); α-hydroxyalkylphenones such as 1-hydroxy-cyclohexyl-phenyl-ketone (commercially available as Omnirad 184 from IGM Resins B.V.); 2-dimethylamino-2-(4-methyl-benzyl)-1-(4 morpholin-4-yl-phenyl)-butan-1-one (commercially available as Omnirad 379 EG from IGM Resins B.V.); 2-benzyl-2-(dimethylamino)-4'-morpholinobutyrophenone (commercially available as Omnirad 369 from IGM Resins B.V.); and the like.

Examples of acylphosphine oxide compounds include, but are not limited to, 2,4,6-trimethylbenzoyl-diphenyl-phosphine oxide (commercially available as Omnirad TPO H from IGM Resins B.V.), bis(2,4,6-trimethylbenzoyl)-phenylphosphine oxide (commercially available as Omnirad 819 from IGM Resins B.V.), and the like.

Other examples of photo-radical polymerization initiators include, but are not limited to, 2-hydroxy-2-methyl-1-phenylpropan-1-one, diethoxyacetophenone, 1-(4-isopropylphenyl)-2-hydroxy-2-methylpropan-1-one, 1-(4-dodecylphenyl)-2-hydroxy-2-methylpropan-1-one, 4-(2-hydroxyethoxy)-phenyl(2-hydroxy-2-propyl)ketone, 2-methyl-1-[4-(methylthio)phenyl]-2-morpholinopropan-1-one, benzoin, benzoin methyl ether, benzoin ethyl ether, benzoin isopropyl ether, benzoin n-butyl ether, benzoin phenyl ether, benzil dimethyl ketal, benzophenone, benzoylbenzoate, methyl benzoylbenzoate, 4-phenylbenzophenone, hydroxybenzophenone, acrylated benzophenone, 4-benzoyl-4'-methyldiphenylsulfide, 3,3'-dimethyl-4-methoxybenzophenone, thioxanthone, 2-chlorothioxanthone, 2-methylthioxanthone, 2,4-dimethylthioxanthone, isopropylthioxanthone, 2,4-dichlorothioxanthone, 2,4-diethylthioxanthone, 2,4-diisopropylthioxanthone, 2,4,6-trimethylbenzoyl diphenylphosphine oxide, methylphenylglyoxylate, benzil, camphorquinone, and the like.

Any one of the photo-radical polymerization initiators may be used alone, or two or more may be used in combination.

From the viewpoint of curing speed and pot life of the curable composition, the content of photo-radical polymerization initiator is preferably 0.01 to 10% by weight, and more preferably 0.04 to 8% by weight of the total weight of the curable composition.

By including a thermal radical polymerization initiator in the curable composition, it is possible to cure the resin composition in a short heating time. The types of thermal radical polymerization initiators are not particularly limited and known materials can be used. Specific examples of thermal radical polymerization initiators include, but are not limited to, dialkyl peroxides such as dicumyl peroxide, *t*-butylcumyl peroxide, 1,3-bis(2-*t*-butylperoxyisopropyl)benzene or 2,5-dimethyl-2,5-bis(t-butylperoxy)hexane; 1,1-bis(*t*-butylperoxy)cyclohexane, 1,1-bis(*t*-butylperoxy)-3,3,5 trimethylcyclohexane, 1,1-bis(*t*-amylperoxy)cyclohexane, 2,2-bis(*t*-butylperoxy)butane; peroxyketals such as *n*-butyl 4,4-bis(*t*-butylperoxy)valerate or ethyl 3,3-(*t-*butylperoxy)butyrate; and alkyl peroxyesters such as t-butylperoxy 2-ethylhexanoate, 1,1,3,3-tetramethylbutylperoxy-2-ethylhexanoate, *t*-butylperoxyisobutyrate, *t-*butylperoxymaleate, or *t*-butylperoxybenzoate. Any one type of thermal radical polymerization initiators may be used alone, or two or more may be used in combination.

When the curable composition contains a thermal radical polymerization initiator, the content of the thermal radical polymerization initiator is preferably 0.01 to 10 % by weight, and more preferably 0.1 to 8 % by weight relative to the total weight of the curable composition.

In one embodiment, the curable composition of the above aspect can further contain a reactive diluent, a solvent, or a combination thereof. This allows the viscosity of the curable composition to be adjusted and improves handling.

The reactive diluent has reactive groups that react with the curable component during curing and are incorporated into the molecule, thus reducing the property degradation of the curable composition to a low level. As a reactive diluent, there is no particular limitation and known materials can be used. From the viewpoint of reactivity with acryloyl groups of dimer acid-modified acrylamide, (meth)acrylate compounds or maleimide compounds having viscosity of 1 Pa-s or less can be preferably used. The reactive diluent can also be added as a medium during the extraction or concentration operations of dimer acid-modified acrylamide.

Examples of (meth)acrylate compounds as a reactive diluent include, but are not limited to, ethyl (meth)acrylate, trifluoroethyl (meth)acrylate, diethylaminoethyl (meth)acrylate, dimethylaminoethyl (meth)acrylate, glycidyl (meth)acrylate, n-butyl (meth)acrylate, isobutyl (meth)acrylate, tert-butyl (meth)acrylate, isoamyl (meth)acrylate, cyclohexyl (meth)acrylate, 1,4-cyclohexanedimethanol mono(meth)acrylate, 2-ethylhexyl (meth)acrylate, isodecyl (meth)acrylate, isobornyl (meth)acrylate, stearyl (meth)acrylate, lauryl (meth)acrylate, phenoxyethyl (meth)acrylate, benzyl (meth)acrylate, tetrahydrofurfuryl (meth)acrylate, ethoxydiethylene glycol (meth)acrylate, phenoxydiethylene glycol (meth)acrylate, phenoxypolyethylene glycol (meth)acrylate, butoxydiethylene glycol (meth)acrylate, methoxydipropylene glycol (meth)acrylate, methoxypolyethylene glycol (meth)acrylate, methoxytriethylene glycol (meth)acrylate, methoxy polyethylene glycol (meth)acrylate, 2-ethylhexyl diethylene glycol (meth)acrylate, 4-tert-butylcyclohexyl(meth)acrylate, 3-phenoxybenzyl(meth)acrylate, 2-hydroxyethyl(meth)acrylate, 2-hydroxypropyl(meth)acrylate, 2-hydroxybutyl(meth)acrylate, 2-hydroxy-3-phenoxypropyl(meth)acrylate, octyl (meth)acrylate, nonyl (meth)acrylate, isononyl (meth)acrylate, 3,3,5-trimethylcyclohexyl (meth)acrylate, cyclic trimethylolpropane formal (meth)acrylate, 1-naphthalene methyl (meth)acrylate, 1-ethylcyclohexyl (meth)acrylate, 1-methylcyclohexyl (meth)acrylate, 1-ethylcyclopentyl (meth)acrylate, 1-methylcyclopentyl(meth)acrylate, dicyclopentenyl(meth)acrylate, dicyclopentenyloxyethyl(meth)acrylate, dicyclopentanyl(meth)acrylate, nonylphenoxy polyethylene glycol(meth)acrylate, tetrahydrodicyclopentadienyl(meth)acrylate, 2-(o-phenylphenoxy)ethyl(meth)acrylate, isobornylcyclohexyl(meth)acrylate, (2-methyl-2-ethyl-1,3-dioxolan-4-yl)methyl(meth)acrylate, 1-adamantyl(meth)acrylate, 3-hydroxy-1-adamantyl(meth)acrylate, 2-methyl-2-adamantanyl(meth)acrylate, 2-ethyl-2-adamantanyl(meth)acrylate, 2-isopropyladamantan-2-yl(meth)acrylate, 3-hydroxy-1-adamantyl(meth)acrylate, (adamantane-1-yloxy)methyl(meth)acrylate, 2-isopropyl-2-adamantyl(meth)acrylate, 1-methyl-1-ethyl-1-adamantylmethanol(meth)acrylate, 1,1-diethyl-1-adamantylmethanol(meth)acrylate, 2-cyclohexylpropan-2-yl(meth)acrylate, 1-isopropylcyclohexyl(meth)acrylate, 1-methylcyclohexyl(meth)acrylate, 1-ethylcyclopentyl(meth)acrylate, 1-methylcyclohexyl(meth)acrylate, tetrahydropyranyl(meth)acrylate, tetrahydro-2-furanyl(meth)acrylate, 2-oxotetrahydrofuran-3-yl(meth)acrylate, (5-oxotetrahydrofuran-2-yl)methyl(meth)acrylate, (2-oxo-1,3-dioxolan-4-yl)methyl(meth)acrylate, 1-ethoxyethyl(meth)acrylate, tricyclodecanedimethanol (meth)acrylate, cyclohexanedimethanol mono(meth)acrylate, dipropylene glycol di(meth)acrylate, 1,6-hexanediol di(meth)acrylate, tripropylene glycol di(meth)acrylate, PO-modified neopentyl glycol di(meth)acrylate, PEG-modified di(meth)acrylate, trimethylolpropane tri(meth)acrylate, trimethylolpropane ethoxy tri(meth)acrylate, glycerol propoxy tri(meth)acrylate, pentaerythritol alkoxytetra(meth)acrylate, pentaerythritol (tri/tetra)(meth)acrylate, and the like. Any one of them may be used alone, or two or more may be used in combination. As used herein, "(meth)acrylate compounds" refer to both acrylate and methacrylate compounds.

Examples of maleimide compounds as a reactive diluent include, but are not limited to, maleimide; maleimides containing aliphatic hydrocarbon groups such as methylmaleimide, ethylmaleimide, propylmaleimide, butylmaleimide, hexylmaleimide, octylmaleimide, dodecylmaleimide, stearylmaleimide and cyclohexylmaleimide; maleimides containing aromatic rings such as phenylmaleimide; and the like. Any one of them may be used alone, or two or more may be used in combination.

Any one of the reactive diluents may be used alone, or two or more may be used in combination.

When the curable composition of this aspect contains a reactive diluent, the content of the reactive diluent can be adjusted according to the desired viscosity, and is, for example, 0 to 99% by weight, for example, 1 to 95% by weight, relative to the total weight of the curable composition.

The solvent can be a medium contained in the composition containing dimer acid-modified acrylamide as mentioned above. Alternatively, depending on the viscosity of the composition required, to the solvent can be selected from organic solvents commonly used in the field of curable compositions, such as, but not limited to, hydrocarbons (benzene, toluene, xylene, cyclohexane, etc.), non-protic polar solvents (N,N-dimethylformamide, dimethyl sulfoxide, N-methyl-2-pyrrolidone, etc.), nitriles (acetonitrile, etc.), ketones (acetone, methyl ethyl ketone, methyl isobutyl ketone, cyclohexanone, etc.), esters (ethyl acetate, butyl acetate, etc.), ethers (cyclopentyl methyl ether, diethyl ether, tetrahydrofuran, dimethoxyethane, etc.), alcohols (methanol, ethanol, propanol, butanol, etc.), terpenes (turpentine oil, turpineol, isobornyl acetate, etc.), halogenated solvents (dichloromethane, chloroform, etc.), and the like.

When the curable composition of this aspect contains a solvent, the content of the solvent can be adjusted according to the desired viscosity, and is, for example, 0 to 60% by weight, for example, 0 to 15% by weight, and for example, 0 to 6% by weight, relative to the total weight of the curable composition.

The curable composition of this aspect may contain a stabilizer to the extent that the effect of the invention is not impaired. Stabilizers are added to enhance the stability of the curable composition during storage and to prevent the occurrence of polymerization reactions caused by unintended radicals or basic components. Typically, stabilizers include radical polymerization inhibitors and anionic polymerization inhibitors.

Known radical polymerization inhibitors can be used. Examples of radical polymerization inhibitors include, but are not limited to, amine-based radical polymerization inhibitors such as N-nitroso-N-phenylhydroxylamine aluminum (NNAS) and phenothiazines; triphenylphosphine; phenol-based polymerization inhibitors such as *p*-methoxyphenol and 2,6-di-*t*-butyl-4-methylphenol; and hydroquinone-based polymerization inhibitors such as hydroquinone, methoxyhydroquinone and di-t-butylhydroquinone. Also, the known radical polymerization inhibitors disclosed in JP 2010-117545 A, JP 2008-184514 A, etc. may be used. Any one of radical polymerization inhibitors may be used alone, or two or more may be used in combination.

When a radical polymerization inhibitor is contained, the content of the radical polymerization inhibitor is preferably from 0.0001 to 5% by weight and more preferably from 0.001 to 3% by weight, relative to the total weight of the curable composition, from the viewpoint of pot life.

Known anionic polymerization inhibitors can be used. Examples thereof include borate compounds and strong acids. Specific examples of anionic polymerization inhibitors include, but not limited to, trimethylborate, triethylborate, tri-n-propylborate, triisopropylborate, trifluoromethanesulfonic acid, maleic acid, methanesulfonic acid, barbituric acid, difluoroacetic acid, trichloroacetic acid, phosphoric acid, and dichloroacetic acid. Anionic polymerization inhibitors can also be used, such as known ones disclosed in JP 2010-117545 A, JP 2008-184514 A, JP 2017-171804 A, and others. Any one of anionic polymerization inhibitors may be used alone, or two or more may be used in combination.

When an anion polymerization inhibitor is contained, the content of the anion polymerization inhibitor is preferably 0.001 to 5% by weight, more preferably 0.01 to 3% by weight, relative to the total weight of the curable composition.

The curable composition of this aspect can contain other curable components other than the above dimer acid-modified acrylamide, to the extent that the characteristics of the curable composition of this aspect are not impaired. Examples of other curable components include, but are not limited to, epoxy compounds (resins), (meth)acrylate compounds (resins), maleimide compounds (resins), acrylamide compounds (resins), vinylethers (resins), styrene derivatives, amine compounds (resins), oxetane compounds (resins), thiol compounds (resins), phenol compounds (resins), and the like. In particular, the inclusion of a maleimide compound (resin) in the curable composition as another curable component enables light curing without using a photo-radical polymerization initiator. Bismaleimide with a dimer acid skeleton can be used as such a maleimide compound (resin).

The curable composition of this aspect may, if desired, contain other additives, such as carbon black, titanium black, a silane coupling agent, an ion trapping agent, a leveling agent, an antioxidant, a defoaming agent, a viscosity modifier, a photosensitizer, a flame retardant, or the like as needed and to the extent that the properties of the curable composition of this aspect are not impaired. The type and amount of each additive are as usual.

The method of producing the curable composition of this aspect is not limited. For example, each component can be introduced simultaneously or separately into an appropriate mixing machine and subjected to stirring and mixing while melting by heating, if necessary, to obtain the curable composition as a uniform composition. The equipment for mixing these materials is not limited. A ricer, Henschel mixer, 3-roll mill, ball mill, planetary mixer, bead mill, and the like, equipped with an agitator and heating device can be used. A combination of these devices may also be used as appropriate.

The curable composition of this aspect can be a one-component curable composition composed as contained in a single container or a two-component (or multi-component) curable composition composed as contained in two or more separate containers, depending on its application, etc. When making a two-component (or multi-component) curable composition, it is conceivable to separate the dimer acid-modified acrylamide and the radical polymerization initiator by placing the dimer acid-modified acrylamide in any container and the radical polymerization initiator in a different container from it. Each container can contain other optional ingredients as needed. An example of such an embodiment is a curable composition kit having a first container containing dimer acid-modified acrylamide and other optional ingredients as needed, and a second container containing a radical polymerization initiator and other optional ingredients as needed.

The curable composition thus obtained is light-curable, thermo-curable, or light and thermo-curable, depending on the type of radical polymerization initiator included. Depending on the application, light curing, thermal curing, or a combination of light and thermal curing can be selected.

When light curing the curable composition, the light irradiated is, for example, ultraviolet (UV) light. In some embodiments, the curable composition may be light cured only. In some embodiments, the curable composition may be further thermally cured after light curing.

When the curable composition is thermally cured, for example, it can be cured by heat treatment at 60 to 200°C for 0.1 to 180 minutes. In some embodiments, the curable composition may be thermally cured only. In some embodiments, thermal curing and light irradiation may be combined.

The method of application of the curable composition of this aspect is not particularly limited, and can be provided, for example, to a desired portion of a substrate or the like by a known printing, dispensing, or coating method. Printing or dispensing methods include, but are not limited to, aerosol jet printing, inkjet printing (jet dispense printing), screen printing, flat plate printing, carton printing, metal printing, offset printing, gravure printing, flexographic printing, and methods using air dispensers. Coating methods include, but are not limited to, dip coating, spray coating, bar coater coating, gravure coating, reverse gravure coating, and spin coater coating.

The curable composition of this aspect can be used, for example, as an adhesive or sealant for fixing, bonding, or protecting components that constitute semiconductor devices or electronic components, or as a raw material thereof. In particular, the curable composition of this aspect can provide a cured product with higher adhesion strength, higher UV curing depth, and higher reliability compared to curable compositions containing bismaleimide with a dimer acid skeleton.

### [Adhesive or sealant]

An adhesive or sealant, which is one aspect of the invention, contains the curable composition of the aspect described above. The adhesive or sealant enables good fixation, bonding, or protection for engineering plastics (e.g., LCP (liquid crystal polymer), polyamide, polycarbonate, etc.), ceramics, and metals (e.g., copper, nickel, etc.). The adhesive or sealant of this aspect can be preferably used to fix, bond, or protect parts comprising a semiconductor device or an electronic component. Examples of the semiconductor device or electronic component include, but are not limited to, HDDs, semiconductor elements, optical sensor modules such as image sensor modules and TOF sensor modules, other semiconductor modules, integrated circuits, and the like.

The adhesive or sealant of this aspect can be a one-component adhesive or sealant composed as contained in a single container, or a two-component (or multi-component) adhesive or sealant composed as contained in two or more separate containers, depending on its application, etc. When making a two-component (or multi-component) adhesive or sealant, it is conceivable to separate the dimer acid-modified acrylamide and the radical polymerization initiator by placing the dimer acid-modified acrylamide in any container and the radical polymerization initiator in a different container from it. Each container can contain other optional ingredients as needed. An example of such an embodiment is an adhesive or sealant kit having a first container containing dimer acid-modified acrylamide and other optional ingredients as needed, and a second container containing a radical polymerization initiator and other optional ingredients as needed.

### [Cured product of the curable composition or adhesive or sealant]

The cured product, which is one aspect of the present invention, is a cured product in which the curable composition of the aspect described above or the adhesive or sealant of the aspect described above has been cured. This cured product is flexible and has excellent adhesive properties. In particular, compared to cured products of curable compositions containing bismaleimide with a dimer acid skeleton, cured products with higher adhesive strength and higher UV curing depth are provided, resulting in superior reliability.

### [Semiconductor device and electronic component]

The semiconductor device or electronic component, which is one aspect of the present invention, contains the cured product of the aspect described above. The term "semiconductor device" refers to all devices that can function by utilizing semiconductor characteristics and includes electronic components, semiconductor circuits, modules incorporating these components, and electronic equipment. Examples of the semiconductor device or electronic component include, but are not limited to, HDDs, semiconductor elements, optical sensor modules such as image sensor modules and TOF sensor modules, other semiconductor modules, integrated circuits, and the like.

### Examples

Hereinbelow, the present invention will be described in more detail with reference to the following Examples and Comparative Examples, which should not be construed as limiting the scope of the present invention. In the following examples, parts and percentages refer to parts by weight and percentages by weight, unless otherwise noted.

### Example A: Production of dimer acid-modified acrylamide

Attempts were made to produce dimer acid-modified acrylamide as follows.

### (Comparative Production Example 1)

In a 200-mL flask under argon gas flow, dimer diamine 1 (trade name "PRIAMINE 1075" from Croda Japan K.K.) (6.00 g), cyclopentyl methyl ether (CPME) (100 mL), and triethylamine (2.27 g) were charged and the mixture was cooled on ice. Acryloyl chloride (2.03 g) was added dropwise thereto at an internal temperature below 15°C. After the drop was completed, the temperature of the mixture was raised to room temperature. 2 hours later, the disappearance of the raw material was confirmed by TLC, and the reaction was terminated. Distilled water (50 mL) and saturated brine (50 mL) were added to the reaction solution, and an attempt was made to wash the dimer acid-modified acrylamide in aliquots, but it became an emulsion and difficult to separate the layers.

### <Discussion>

Normally, CPME can be used as a reaction solvent and extraction solvent, but in this comparative production example, the target product could not be separated due to difficulties in fractionation. This is probably due to the amphiphilic nature of dimer acid-modified acrylamide.

### (Production Example 1)

In a 100-mL flask under argon gas flow, dimer diamine 1 (trade name "PRIAMINE 1075" from Croda Japan K.K.) (6.00 g), cyclopentyl methyl ether (CPME) (60 mL) and triethylamine (2.27 g) were charged and the mixture was cooled on ice. Acryloyl chloride (2.03 g) was added dropwise thereto at an internal temperature below 15°C. After the drop was completed, the temperature of the mixture was raised to room temperature. 2 hours later, the disappearance of the raw material was confirmed by TLC, and the reaction was terminated.

Distilled water (10 mL), 1M HCl (2.5 mL), and ethyl acetate (30 mL) were added to the reaction solution. The organic layer was separated and the remaining layer was extracted with ethyl acetate (30 mL). The organic layers were combined and saturated brine (10 mL) and 1M NaOH (1 mL) were added thereto. The organic layer was separated and the remaining layer was extracted with ethyl acetate (15 mL). The organic layers obtained were combined, checked for neutrality, dried with sodium sulfate, filtered, and concentrated in an evaporator in a hot water bath at 45°C to obtain 7.58 g of a composition (A-1) containing dimer acid-modified acrylamide. ¹H NMR showed that about 15% by weight of CPME and 0.5% by weight of ethyl acetate remained in the composition at this stage. The composition (A-1) does not contain any polymerization inhibitor.

### <Discussion>

Compared to Comparative Production Example 1, by devising an extraction solvent, a dimer acid-modified acrylamide composition containing about 15% by weight of medium could be obtained. More specifically, after intensive study, it was surprisingly found that ethyl acetate, which has lower solubility of dimer-acid modified acrylamide than CPME and higher affinity with water than CPME, is a better extraction solvent for dimer acid-modified acrylamide. The detailed reason is unknown and is not limited to this, but it is thought that the carbonyl group of ethyl acetate interacts with the NH group of acrylamide to prevent micelle formation by dimer acid-modified acrylamide.

### (Comparative Production Example 2)

Methylhydroquinone (MEHQ) (1.5 mg (equivalent to 200 ppm)) was added as a polymerization inhibitor to the composition (A-1) of Production Example 1 (about 7 g), and the solvent was further removed at 50°C under high vacuum. This resulted in the composition becoming highly viscous in about 2 hours and the formation of a gel insoluble in chloroform.

### <Discussion>

This could be, but is not limited to, an unexpected radical generation and partial polymerization in an environment with significantly reduced oxygen levels.

### (Production Example 2)

In a 10L flask under argon gas flow, dimer diamine 1 (trade name "PRIAMINE 1075" from Croda Japan K.K.) (600g), cyclopentyl methyl ether (CPME) (6L), triethylamine (227.7g) were charged and the mixture was cooled on ice. Acryloyl chloride (203.7 g) was added dropwise thereto at an internal temperature of 15°C or lower. After the drop was completed, the temperature of the mixture was raised to room temperature. 2 hours later, the disappearance of the raw material was confirmed by TLC, and the reaction was terminated.

Distilled water (1L), 1M HCl (250mL), and ethyl acetate (4L) were added to the reaction solution. The organic layer was separated and the remaining layer was extracted with ethyl acetate (2L). The organic layers were combined and saturated brine (1L) and 1M NaOH (300mL) were added thereto. The organic layer was separated and the remaining layer was extracted with ethyl acetate (1.5 L). The resulting organic layers were combined, checked for neutrality, dried with sodium sulfate, filtered, and phenothiazine (65.9 mg (equivalent to about 100 ppm)) was added to the organic layer, then concentrated in an evaporator in a 35°C water bath to obtain 729 g of dimer acid-modified acrylamide composition (A-2) containing about 89.9% by weight of dimer acid-modified acrylamide, about 10% by weight of CPME, and about 0.1% by weight of ethyl acetate. The composition was soluble in chloroform, and the content of CPME and ethyl acetate was calculated from the integral of ¹H NMR measured in deuterated chloroform.

### <Discussion>

In this production example, a scale-up was attempted for Production Example 1, and a polymerization inhibitor was used during concentration. A dimer acid-modified acrylamide composition containing about 10% by weight of medium, with relatively high fluidity and easy handling, was obtained.

### (Production Example 3)

About 70 g of dimer acid-modified acrylamide composition (A-2) prepared according to Production Example 2 was further concentrated at 45°C under high vacuum to obtain 66 g of dimer acid-modified acrylamide composition (A-3) containing about 5% by weight of CPME. The composition is soluble in chloroform, and the CPME content was calculated from the integral of ¹H NMR.

### <Discussion>

It is believed that, unlike Comparative Production Example 2, since the polymerization inhibitor was effective even in an environment with low oxygen concentration, a dimer acid-modified acrylamide composition containing about 5% by weight of medium could be obtained without gel formation even after concentration under high vacuum.

### (Production Example 4)

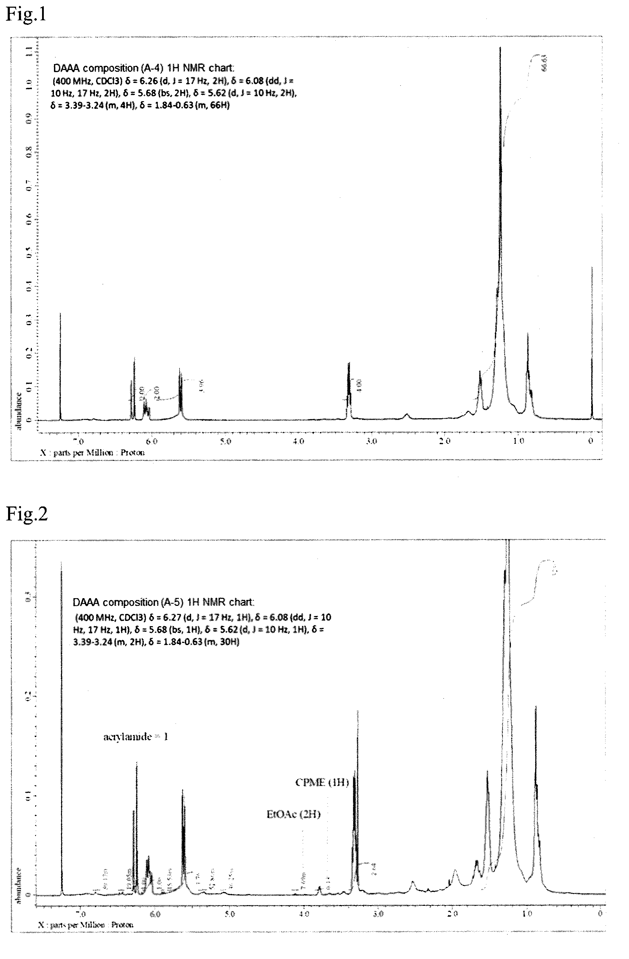

About 14 g of dimer acid-modified acrylamide composition (A-2) prepared according to Production Example 2 was charged in a beaker and dried in a vacuum dryer at 60°C for 8 hours to obtain 12.6 g of dimer acid-modified acrylamide composition (A-4). The composition was soluble in chloroform, and no solvents such as CPME were detected by ¹H NMR. A ¹H NMR chart of dimer acid-modified acrylamide composition (A-4) is shown in Figure 1.

### <Discussion>

By using an appropriate polymerization inhibitor, a composition was obtained that contained dimer acid-modified acrylamide and a polymerization inhibitor and was substantially free of medium-.

### (Production Example 5)

In a 300-mL flask under argon gas flow, dimer diamine 2 (trade name "PRIAMINE 1071" from Croda Japan K.K.) (18.0 g), cyclopentyl methyl ether (CPME) (180 mL), and triethylamine (6.90 g) were added and cooled on ice. Acryloyl chloride (6.17 g) was added dropwise to the mixture at an internal temperature below 15°C. After the drop was completed, the temperature of the mixture was raised to room temperature. After 2 hours, the disappearance of the raw material was confirmed by TLC and the reaction was terminated.

The reaction solution was cooled on ice and distilled water (30 mL), 1M HCl (7.5 mL), and ethyl acetate (90 mL) were added thereto. The organic layer was separated and the remaining layer was extracted with ethyl acetate (90 mL). The organic layers were combined and saturated brine (30 mL) and 1M NaOH (3 mL) were added thereto. The organic layer was separated and the remaining layer was extracted with ethyl acetate (45 mL). The resulting organic layers were combined, checked for neutrality, dried with sodium sulfate, filtered, and concentrated in an evaporator in a hot water bath at 45°C to obtain 17.0 g of dimer acid-modified acrylamide composition (A-5) containing about 0.1% by weight of ethyl acetate and 3.9% by weight of CPME. The composition was soluble in chloroform, and the content of ethyl acetate and CPME was calculated from the integral of ¹H NMR. A ¹H NMR chart of dimer acid-modified acrylamide composition (A-5) is shown in Figure 2.

### (Production Example 6)

To 8.0 g of dimer acid-modified acrylamide composition (A-5) of Production Example 5, N-nitroso-N-phenylhydroxylamine aluminum (NNAS) (equivalent to 0.16 mg) diluted in ethyl acetate was added and mixed well, then the solvent was removed under high vacuum at 45°C to obtain 8.0 g of dimer acid-modified acrylamide composition (A-6) containing about 20 ppm of NNAS, about 0.2% by weight of ethyl acetate and 3.3% by weight of CPME. The composition was soluble in chloroform, and the content of ethyl acetate and CPME was calculated from the integral of ¹H NMR.

### [¹H NMR and LC-MS analysis of dimer acid-modified acrylamide]

¹H NMR and LC-MS analysis of dimer acid-modified acrylamide compositions obtained in the above production examples was performed.

¹H NMR measurement examples:
Production Example 4 (A-4): 1H NMR (400 MHz, CDCl3) δ = 6.26 (d, J = 17 Hz, 2H), δ = 6.08 (dd, J = 10 Hz, 17 Hz, 2H), δ = 5.68 (bs, 2H), δ = 5.62 (d, J = 10 Hz, 2 H), δ = 3.39-3.24 (m, 4H), δ = 1.84-0.63 (m, 66H). See Figure 1 for chart.
Production Example 5 (A-5): 1H NMR (400 MHz, CDCl3) δ = 6.27 (d, J = 17 Hz, 1H), δ = 6.08 (dd, J = 10 Hz, 17 Hz, 1H), δ = 5.68 (bs, 1H), δ = 5.62 (d, J = 10 Hz, 1H), δ = 3.39-3.24 (m, 2H), δ = 1.84-0.63 (m, 30H). See Figure 2 for chart. The LC-MS analysis conditions are as follows.
Mass spectrometer: Orbitrap Fusion Lumos (Thermo Fisher Scientific Co. Ltd)
Columns: Kinetex 2.6 µm XB-C18 100Å (phenomenex) 50 × 3.0 mm
Mobile phase A: Water
Mobile phase B: Methanol
Time-Program: 90%B(0.00min) - 100%B(15min) - STOP (22min)
Flow rate: 0.3 mL/min
Column Temp.: 40°C
Ionization mode: ESI
Probe voltage: +3.5 kV (ESI-positive mode)
Ion transfer tube temp.: 300 °C
Vaporizer temp.: 350 °C
Mass range(m/z): 150-1600

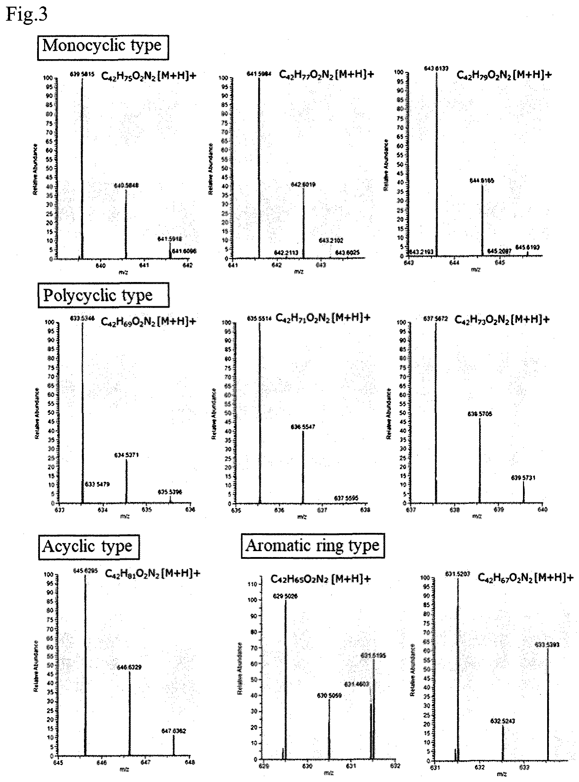

An example of the mass spectrum obtained by LC-MS analysis of dimeric acid-modified acrylamide obtained in Production Example 4 is shown in Figure 3.

¹H NMR analysis and LC-MS analysis estimated that the dimer acid-modified acrylamide obtained in Production Example 4 contains diacrylamide compounds with the following structures.

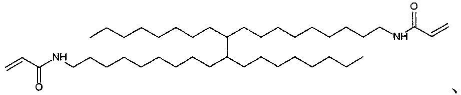

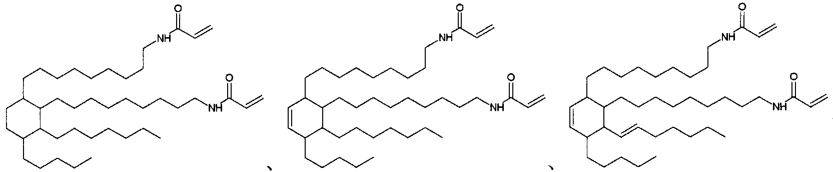

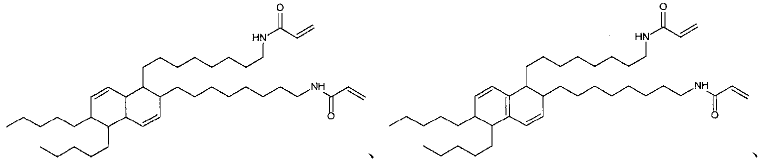

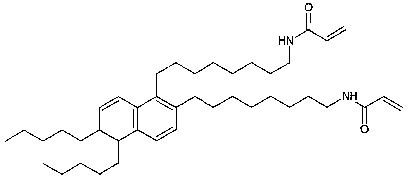

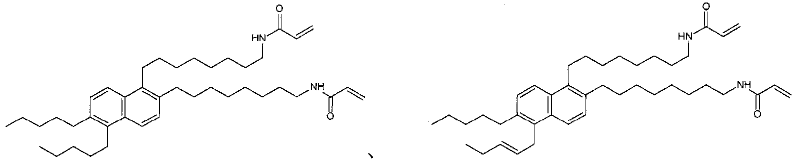

### [Viscosity Measurement]

The viscosities of the dimer acid-modified acrylamide compositions of the above production examples were measured using a DV-I Prime digital viscometer at 25°C and a rotation speed of 5 rpm. The results are shown in Table 1.

**[Table 1]**

| | Medium content (wt%) | Viscosity[Pa · s] |
|---|---|---|
| Production Example 2 | 10 | 16 |
| Production Example 3 | 5 | 56 |
| Production Example 4 | <0.5 | 110 |

The viscosities of the dimer acid-modified acrylamide compositions of Production Examples 2 and 3 were lower than the viscosity of Production Example 4, which contains substantially no medium, the viscosity of the medium, and the viscosity (calculated mixing viscosity) assumed from the formulation of the composition.

The viscosity of the dimer acid-modified acrylamide composition of Production Example 4, which contains substantially no medium, is high, and there is concern that uniform mixing of the components may become difficult and air bubbles may be entrapped if it is used in the preparation of the curable composition. Therefore, in order to avoid these problems, it is found that it is preferable to adjust the viscosity of the dimer acid-modified acrylamide composition by using a reactive diluent, a solvent, or a combination thereof, as appropriate, when preparing the curable composition.

For greater flexibility in formulating curable compositions and to reduce volatile organic compounds (VOCs), the lower the medium content in the composition, the better.

### Example B: Production and properties evaluation of curable compositions

### [Examples 1 to 13, Comparative Examples 1 to 10]

Curable compositions were prepared by mixing predetermined amounts of each component according to the formulations shown in Tables 2 to 4. In Tables 2 to 4, the amount of each component is expressed in parts by weight (unit: g). The components used in the examples and comparative examples are as follows.

### - (A) Dimeric acid-modified acrylamide

(A-3): Dimeric acid-modified acrylamide composition (A-3) of the above Production Example 3 was used. The amount of the component (A-3) listed in Table 1 is the amount as a composition including medium and polymerization inhibitor.
(A-5): Dimeric acid-modified acrylamide composition (A-5) of the above Production Example 5 was used. The amount of the component (A-5) listed in Table 1 is the amount as a composition including medium and polymerization inhibitor.
(A-6): Dimeric acid-modified acrylamide composition (A-6) of the above Production Example 3 was used. The amount of the component (A-6) listed in Table 1 is the amount as a composition including medium and polymerization inhibitor.

### - (A') Dimeric acid-modified maleimide

(A'-1): Dimer bismaleimide containing mainly 1,1'-((4-hexyl-3-octylcyclohexan-1,2-diyl) bis(octan-8,1-diyl) bis(1H-pyrrol-2,5-dione) (trade name: BMI-689, from Designer Molecules)

### - (B) Photo-radical polymerization initiator

(B-1): 1-hydroxy-cyclohexyl-phenyl-ketone (trade name: Omnirad 184, from IGM Resins)

### - (C) Thermal radical polymerization initiator

(C-1): 1,1,3,3-tetramethylbutylperoxy-2-ethylhexanoate (trade name: Perocta-O, from NOF CORPORATION)

### - (D) Reactive diluent

(D-1): Isobornyl acrylate (trade name: IBXA, from Kyoeisha Chemical Co., Ltd.)
(D-2): 1,4-Cyclohexanedimethanol monoacrylate (trade name: CHDMMA, manufactured by Nihon Kasei CO., LTD)
(D-3): Dimethylol-tricyclodecanediacrylate (trade name: Light Acrylate DCP-A, from Kyoeisha Chemical Co., Ltd.)

In the examples and comparative examples, the properties of the curable compositions were measured as follows.

### [UV curing depth measurement]

The curable compositions of Examples 1 to 3 and Comparative Example 1, as well as Examples 9 to 14 and Comparative Examples 7 to 9, were defoamed in a vacuum, applied to a LCP (LAPEROS^{®} E463i) plate at 0.3 mm thickness, and fixed by sandwiching it with another LCP plate. Specimens were prepared by irradiating UV from the side of the LCP plates with a UV irradiation dose of 2000 mJ/cm² (UV wavelength: 365 nm, LED lamp) to cure the compositions (n = 6). The UV curing depth was determined by measuring the curing distance of this specimen from the UV irradiated surface to the vertical direction using a measuring microscope. The results are shown in Tables 2 and 4.

### [Adhesive strength of cured product]

### (1) Light curing

The curable compositions obtained in Examples 1 to 3 and Comparative Example 1 were stencil printed on an FR4 substrate with a size of φ2 mm and a thickness of 125 µm. An alumina chip of 1.5 mm x 3 mm x 0.5 mm was placed over the printed resin composition with the 1.5 mm x 3 mm side down to prepare the test specimens (n = 10). The curing conditions were as follows: The UV LED irradiation device AC475 available from Excelitas Technologies Corp. was used to irradiate UV light at an integrated light intensity of 2000 mJ/cm² (measured with UIT-250 (connected to UVD-365 light receiver) available from USHIO Inc.) for curing. The alumina chip on the substrate was poked from the side with MODEL-1605HTP strength tester from Aikoh Engineering Co., Ltd., and the adhesive strength (shear strength) was calculated from the value when the alumina chip peeled off. Using the same procedure, test specimens were prepared using glass and nickel-plated substrates, and adhesive strength (shear strength) was measured. The results are shown in Table 2.

### (2) Thermal curing

The curable compositions obtained in Examples 4 to 8 and Comparative Examples 2 to 6 were stencil printed on a nickel-plated substrate with a size of φ2 mm and a thickness of 125 µm. An alumina chip of 1.5 mm x 3 mm x 0.5 mm was placed over the printed resin composition with the 1.5 mm x 3 mm side down to prepare the test specimens (n = 10). The curing conditions were as follows: the test specimens were heated in an air dryer at 120°C for 60 minutes for curing. The alumina chip on the substrate was poked from the side with MODEL-1605HTP strength tester from Aikoh Engineering Co., Ltd., and the adhesive strength (shear strength) was calculated from the value when the alumina chip peeled off.

**[Table 2]**

| | | | Example 1 | Example 2 | Example 3 | Com. Ex. 1 |
|---|---|---|---|---|---|---|
| (A)Dimeric acid-modified acrylamide | (A-3) | | 95 | | | |
| | (A-5) | | | 95 | | |
| | (A-6) | | | | 95 | |
| (A')Dimeric acid-modified maleimide | (A'-1) | BMI-689 | | | | 95 |
| (B)Photo-radical polymerization initiator | (B-1) | OMNIRAD 184 | 5 | 5 | 5 | 5 |
| (C)Thermal radical polymerization initiator | (C-1) | PEROCTA O | | | | |
| (D)Reactive diluent | (D-1) | IBXA | | | | |
| | (D-2) | CHDMMA | | | | |
| | (D-3) | DCPA | | | | |
| Total | | | 100 | 100 | 100 | 100 |
| UV curing depth (mm) | | | 0.79 | 0.60 | 0.59 | 0.27 |
| UV curing-adhesive strength (N/chip) | Nickel plated substrate / Alumina chip | | 56 | 24 | 28 | 18 |
| UV curing-adhesive strength (N/chip) | FR4 substrate/Alumina chip | | 58 | 40 | 38 | 23 |
| UV curing-adhesive strength (N/chip) | Glass substrate/Alumina chip | | 76 | 88 | 84 | 57 |

As can be seen from Table 2, the curable compositions containing dimer acid-modified acrylamide in the Examples showed better curing depth and adhesive strength in light curing compared to the curable compositions containing dimer acid-modified maleimide in the Comparative Example.

**[Table 3-1]**

| | | Example 4 | Example 5 | Example 6 | Example 7 | Example 8 |
|---|---|---|---|---|---|---|
| (A)Dimeric acid-modified acrylamide | (A-3) | | 45 | 83 | 45 | 83 |
| | (A-5) | 95 | | | | |
| | (A-6) | | | | | |
| (A')Dimeric acid-modified maleimide | (A'-1) | | | | | |
| (B)Photo-radical polymerization initiator | (B-1) | | | | | |
| (C)Thermal radical polymerization initiator | (C-1) | 5 | 5 | 5 | 5 | 5 |
| (D)Reactive diluent | (D-1) | | 50 | 13 | | |
| | (D-2) | | | | 50 | 13 |
| | (D-3) | | | | | |
| Total | | 100 | 100 | 100 | 100 | 100 |
| Thermal curing-adhesive strength (N/chip) (Nickel plated substrate / Alumina chip) | | 65 | 88 | 78 | 122 | 90 |

**[Table 3-2]**

| | | Com.Ex.2 | Com.Ex.3 | Com.Ex.4 | Com.Ex.5 | Com.Ex.6 |
|---|---|---|---|---|---|---|
| (A)Dimeric acid-modified acrylamide | (A-3) | | | | | |
| | (A-5) | | | | | |
| | (A-6) | | | | | |
| (A')Dimeric acid-modified maleimide | (A'-1) | 95 | 44 | 82 | 44 | 82 |
| (B)Photo-radical polymerization initiator | (B-1) | | | | | |
| (C)Thermal radical polymerization initiator | (C-1) | 5 | 5 | 5 | 5 | 5 |
| (D)Reactive diluent | (D-1) | | 51 | 13 | | |
| | (D-2) | | | | 51 | 13 |
| | (D-3) | | | | | |
| Total | | 100 | 100 | 100 | 100 | 100 |
| Thermal curing-adhesive strength (N/chip) (Nickel plated substrate / Alumina chip) | | 40 | 73 | 65 | 112 | 73 |

As can be seen in Table 3, also in heat curing, the curable compositions containing dimer acid-modified acrylamide in the Examples showed better adhesive strength than the curable compositions containing dimer acid-modified maleimide in the Comparative Examples. This is thought to be due to improved adhesion to the substrate due to NH groups in dimer acid-modified acrylamide.

**[Table 4]**

| | | Ex.9 | Ex.10 | Ex.11 | Com.Ex.7 | Com.Ex.8 | Com.Ex.9 | Ex.12 | Ex.13 | Com.Ex.10 |
|---|---|---|---|---|---|---|---|---|---|---|
| (A)Dimeric acid-modified acrylamide | (A-3) | 10 | 10 | 10 | | | | 10 | 50 | |
| | (A-5) | | | | | | | | | |
| | (A-6) | | | | | | | | | |
| (A ')Dimeric acid-modified maleimide | (A'-1) | | | | 10 | 10 | 10 | 90 | 50 | 100 |
| (B)Photo-radical polymerization initiator | (B-1) | 5 | 3 | 1 | 5 | 3 | 1 | | | |
| (C)Thermal radical polymerization initiator | (C-1) | | | | | | | | | |
| (D)Reactive diluent | (D-1) | | | | | | | | | |
| | (D-2) | | | | | | | | | |
| | (D-3) | 85 | 87 | 89 | 85 | 87 | 89 | | | |
| Total | | 100 | 100 | 100 | 100 | 100 | 100 | 100 | 100 | 100 |
| UV curing depth (mm) | | 1.87 | 2.00 | 2.04 | 1.39 | 1.42 | 0.94 | 0.31 | 0.34 | 0.25 |

As can be seen from Table 4, in the case of curable compositions containing dimer acid-modified acrylamide, the UV curing depth did not decrease when the amount of photo-radical polymerization initiator was reduced to 5%, 3%, or 1% by weight (Examples 9 to 11), but in the case of curable compositions containing dimer acid-modified maleimide, the UV curing depth was significantly reduced when the amount of radical polymerization initiator was reduced to 1% by weight (Comparative Examples 7 to 9).

Since light irradiation to maleimide generates radicals through [2+2] cycloaddition reactions, curing is possible with UV irradiation even without the addition of photo-radical initiators. Even in that case, the UV curing depth could be improved by replacing some of the maleimide with dimer acid-modified acrylamide (Examples 12 and 13, and Comparative Example 10).

The disclosure of Japanese Patent Application No. 2023-060069 (filing date: April 3, 2023) is incorporated herein by reference in its entirety.

All references, patent applications, and technical standards described herein are incorporated herein by reference to the same extent as if the individual references, patent applications, and technical standards were specifically and individually noted as being incorporated by reference.

## Claims

1. Dimer acid-modified acrylamide.

2. A composition comprising
dimer acid-modified acrylamide, and
a medium, a polymerization inhibitor, or a combination thereof,
wherein a content of the dimer acid-modified acrylamide in the composition is 83% by weight or more.

3. A curable composition comprising
dimer acid-modified acrylamide, and
a radical polymerization initiator.

4. The curable composition according to claim 3, wherein the radical polymerization initiator is a photo-radical polymerization initiator.

5. The curable composition according to claim 3, wherein the radical polymerization initiator is a thermal radical polymerization initiator.

6. The curable composition according to any one of claims 3 to 5, further comprising a reactive diluent, a solvent, or a combination thereof.

7. The curable composition according to any one of claims 3 to 6, further comprising a curable component other than the dimer acid-modified acrylamide.

8. The curable composition according to any one of claims 3 to 7, which is configured as the dimer acid-modified acrylamide and the radical polymerization initiator being contained in a single container.

9. The curable composition according to any one of claims 3 to 7, which is configured as the dimer acid-modified acrylamide and the radical polymerization initiator being separated in two or more containers.

10. An adhesive or sealant comprising the curable composition according to any one of claims 3 to 9.

11. A cured product in which the curable composition according to any one of claims 3 to 9 or the adhesive or sealant according to claim 10 has been cured.

12. A semiconductor device or an electronic component comprising the cured product according to claim 11.
